# EUROPEAN PATENT SPECIFICATION

(11) **EP 2 468 726 B1**
(45) Date of publication and mention of the grant of the patent: **28.08.2013**
(21) Application number: 10193751.4
(22) Date of filing: 06.12.2010
(51) Int. Cl.: C07D 213/26, C07D 401/10, A61K 31/4418, A61P 35/00, A61P 35/02, A61P 35/04

(54) **Compound for the treatment of tumours and tumour metastases**
Verbindung zur Behandlung von Tumoren und Tumormetastasen
Composant pour le traitement des tumeurs et métastases de tumeurs

(43) Date of publication of application: 27.06.2012
(73) Proprietor: Siena Biotech S.p.A., 53100 Siena (IT)
(72) Inventor: Pericot Mohr, Gal.la, 53100 Siena (IT); Thomas, Russel J., 53100 Siena (IT); Minetto, Giacomo, 53100 Siena (IT); Bellini, Marta, 53100 Siena (IT); Wiedenau Paul H., 53100 Siena (IT); Betti, Matteo, 53100 Siena (IT)
(74) Representative: Banfi, Paolo

(56) References cited:
- WO-A2-2009/074300

## Description

The invention relates to a novel, brain penetrant, Smoothened receptor ligand which antagonises the Hedgehog pathway, to its pharmaceutical applications, to processes for obtaining this compound and to novel intermediates useful in these processes.

### BACKGROUND TO THE INVENTION.

The inhibition of Hedgehog pathway by Smoothened receptor (Smo) antagonists is now a well known approach to treat a variety of cancer types: compounds known as GDC449, LDE225, IPI926 and XL139 are undergoing clinical trials in various cancer settings (an overview of these trials is available from www.clinicaltrials.gov).

Moreover, the peer-reviewed scientific literature is laden with evidence supporting the wide applicability of compounds having Smo antagonising activity in the following, more specific, cancer settings: brain cancers such as medulloblastoma (Romer and Curran, Cancer Res 65(12) 4975 - 4978 (2005)) and glioblastoma (Bar et al. Stem Cells 25(10):2524-33 (2007)); prostate cancer (Sanchez et al. PNAS 101(34) 12561-12566 (2004)); pancreatic cancer (Thayer et al. Nature 423 851 - 856 (2003)); non-small cell lung carcinoma (Yuan et al. Oncogene 26 1046-1055 (2007); small-cell lung cancer (Watkins et al. Nature 422 313-317 (2003)); breast cancer (Kubo et al. Cancer Res 64 6071-6074 (2004)); various digestive tract tumours (Berman et al. Nature 425 846-851 (2003)) and (Lees et al. Gastroenterology 129(5) 1696-1710 (2006)); basal cell carcinoma (Williams et al. PNAS 100(8) 4616-4621 (2003)) and Gorlin syndrome (Epstein et al., Nature Reviews in Cancer, 8, 743, 2008); malignant melanoma (Pons and Quintanilla Clin Trans Oncol. 8(7) 466-474 (2006)); squamous cell carcinomas (Xuan et al. Mod Pathol. 19(8) 1139-47 (2006)); B-cell malignancies such as multiple myeloma and lymphomas (Dierks et al. Nat. Med. 13(8) 944-951 (2007); Peacock et al. PNAS 104(10) 4048-4053 (2007)); mesenchymal cancers such as chondrosarcoma (Tiet et al. Am. J. Pathol. 168(1) 321-330 (2006)), clear cell sarcoma of the kidney (Cutcliffe et al. Clin Cancer Res. 11(22):7986-94 (2005)) and rhabdomyosarcoma (Tostar et al. J. Pathol. 208(1) 17-25 (2006)); chronic myeloid leukaemia (Sengupta et al. Leukemia 21(5) 949-955 (2007)); endometrial carcinoma (Feng et al. Clin. Cancer Res. 13(5) 1389-1398 (2007); hepatocellular carcinomas (Huang et al. Carcinogenesis 27(7) 133401340 (2006)); ovarian tumours (Chen et al. Cancer Sci. 98(1) 68-76 (2007)).

With regard to brain tumours, it is known that the efficacy of an antitumour agent can also be dependent on the ability of the agent to cross the so called blood-brain-barrier (BBB), a complex biological system that protects the brain cells from entering into contact with a large number of substances circulating in the bloodstream. This enhanced efficacy is attributable to the antitumour agent reaching a sub-population of invasive tumour cells which are otherwise protected by the BBB. (Ehtesham et al.,Oncogene, 26,5721,2007 and calabrese e tal., Cancer Cell, 11,69,2007).

It is also known in the field of Oncology that most primary tumours metastasise to secondary *loci*, and that many primary cancer types are likely to metastasise to the brain. In many cases, a cancer diagnosis is only made after the primary tumour has spread to and is detectable in the brain (Agazzi et al., Acta NeuroChirurgica, 146(2), 153-157, 2004). Large autopsy studies suggest that between 20% and 40% of all patients with metastatic cancer will have brain metastases (Weil et al., American Journal of Pathology.;167, 913-920,2005).

The frequency of metastatic brain tumors is thought to be rising due to longer survival after primary cancer diagnosis, which is a direct result of earlier detection and more effective treatment. (Barnholtz-Sloan et al., J. Clin. Oncology, 22,2865(2004)). Individuals with primary lung, breast, skin, or GI tract tumours account for the majority of people diagnosed with brain metastases: in 2700 cases from the Memorial Sloan-Kettering Cancer Center in New York, the distribution of primary cancers was as follows: 48% lung, 15% breast, 9% melanoma, 1% lymphoma (mainly non-Hodgkin), 3% GI (3% colon and 2% pancreatic), 11% genitourinary (21% kidney, 46% testes, 5% cervix, 5% ovary), 10% osteosarcoma, 5% neuroblastoma, and 6% head and neck tumor.

Under this perspective, the treatment of a tumour is seen to also involve the treatment of metastases that may originate from this tumour. Given the above described high incidence of brain metastases, an increased ability of the antitumour agent to cross the blood-brain-barrier is an advantage.

### PRIOR ART

Patent applications WO2006028958 and WO2009126863 disclose pyridyl derivatives which are Smo receptor ligands inhibitors of the Hedgehog pathway useful for the treatment of cancer.

Patent application WO2009074300, in the name of the same applicant, discloses benzimidazole derivatives as Smo receptor antagonists for the treatement of cancer. In particular, this patent discloses compounds A and B.

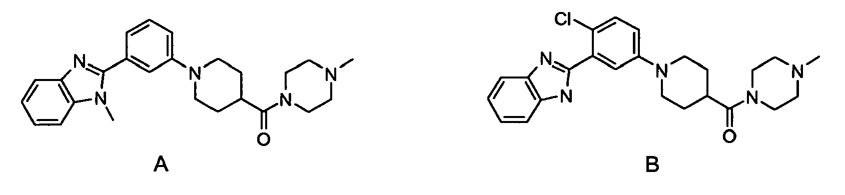

### DESCRIPTION OF THE INVENTION

It has surprisingly been found that compound **C,** which is a Smo receptor antagonist as set out in example 2, has exceptionally high brain permeation properties, particularly with respect to two of its close analogues **A** and **B** disclosed in WO2009074300, as set out in example 3.

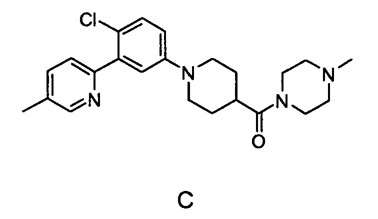

In one embodiment, there is provided compound **C** and pharmaceutically acceptable salts thereof.

In another embodiment, there is provided compound **C** for use as a medicament.

In a particular embodiment, there is provided compound **C** for use in the treatment of cancer, particularly for the treatment of a cancer selected from the list of; non-small cell lung carcinoma; small-cell lung cancer; breast cancer; ovarian tumours; digestive tract tumours; brain cancers; prostate cancer; pancreatic cancer; basal cell carcinoma; Gorlin syndrome; malignant melanoma; squamous cell carcinomas; multiple myeloma; lymphoma; mesenchymal cancers;chronic myeloid leukaemia; endometrial carcinoma; hepatocellular carcinoma.

In a further embodiment, there is provided compound **C** for use in the treatment of brain cancers.

In a yet further embodiment, there is provided compound **C** for use in the treatment of cancer metastases in the brain.

In another embodiment, there is provided compound **C** for use in the treatment of a cancer that metastasises to the brain.

Also described is compound **C** for use as a Smo receptor antagonist.

In another embodiment, there is provided a pharmaceutical composition comprising compound **C** or pharmaceutically acceptable salts thereof, a pharmaceutically acceptable carrier and/or a pharmaceutically acceptable auxiliary substance.

Another embodiment of this invention relates to the use of compound **C** for the manufacture of a medicament, particularly for the manufacture of a medicament to treat cancer.

Another embodiment of this invention relates to the use of compound **C** for the manufacture of a medicament to treat a cancer selected from the list of: non-small cell lung carcinoma; small-cell lung cancer; breast cancer; ovarian tumours; digestive tract tumours; brain cancers; prostate cancer; pancreatic cancer; basal cell carcinoma; Gorlin syndrome; malignant melanoma; squamous cell carcinomas; multiple myeloma; lymphoma; mesenchymal cancers;chronic myeloid leukaemia; endometrial carcinoma; hepatocellular carcinomaOther embodiments of this invention relate to methods of treatment of diseases, conditions or dysfunctions that benefit from the inhibition of the hedgehog pathway, which methods comprise administering to a subject in need thereof an effective amount of compound **C.**

The dosage of compound **C** for use in therapy may vary depending upon, for example, the administration route, the nature and severity of the disease. In general, an acceptable pharmacological effect in humans may be obtained with daily dosages ranging from 0.01 to 200 mg/kg.

The pharmaceutical compositions of the invention can be in the form of solid, semi-solid or liquid preparations, preferably in form of solutions, suspensions, powders, granules, tablets, capsules, syrups, suppositories, aerosols or controlled delivery systems. The compositions can be administered by a variety of routes, including oral, transdermal, subcutaneous, intravenous, intramuscular, rectal and intranasal, and are preferably formulated in unit dosage form. Oral unit dosage forms may contain from about 1 mg to about 1000 mg of the compound of the invention.

This invention also includes acid addition salts of compound **C,** preferably salts with pharmaceutically acceptable acids.

Compound **C** can be obtained starting from novel key intermediate **D** - which is a further embodiment of the invention - as outlined in scheme 1 below wherein "LG" is a suitable leaving group, and as described in full details in example 1, or variants thereof that are within the competence of the average skilled person.

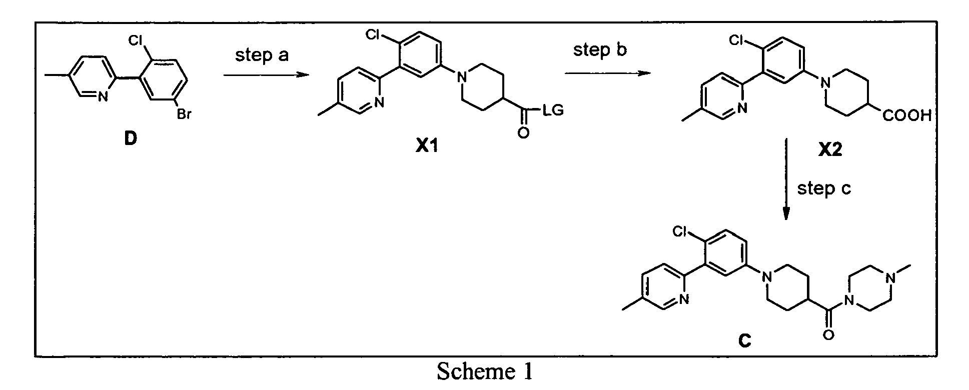

In a particular embodiment, LG is a linear branched or cyclic C₁₋₆ alkoxy group.

Accordingly, there is provided a method for obtaining compound **C** that comprises the use of compound **D** as an intermediate or starting material.

More specifically, there is provided a method for obtaining compound **C** which comprises the steps of:
**a)** Treating compound **D** with at least 1eq of a suitable isonipecotate derivative, under palladium catalysis and in presence of a suitable base so as to obtain compound **X1**
**b)** Converting compound **X1** into compound **X2**
**c)** Coupling compound **X2** with N-methyl-piperazine so as to obtain compound **C.**

Examples on how to perform steps b) and c) above not limitedly include those described by March (in "Advanced Organic Chemistry: Reactions, Mechanisms, and Structure", Sixth Edition, Ed Wiley, ISBN 9780471720911).

Compound **D** can be obtained starting from commercially available compounds using the two alternative synthetic routes outlined in scheme 2 below and described in full details in example 1, or variants thereof that are within the competence of the average skilled person.

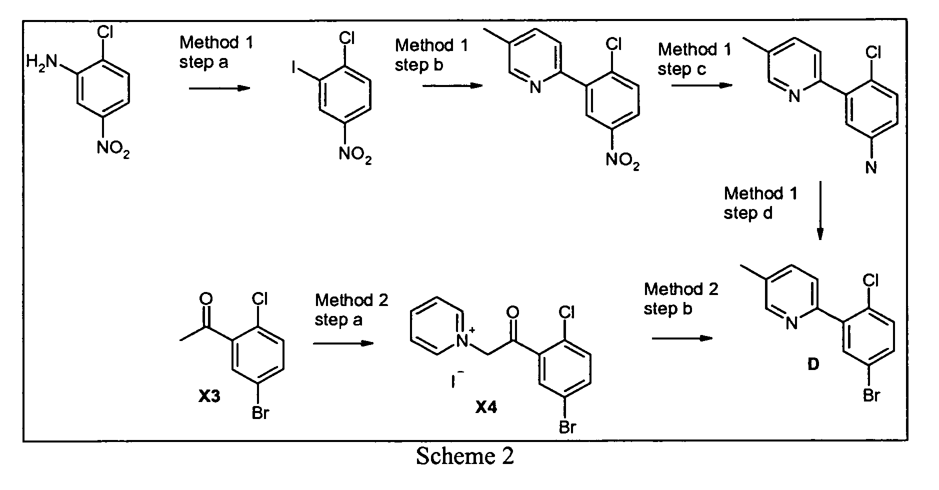

The main drawback of the longest route to intermediate **D** ("method 1" in scheme 2) lies in the high cost of 5-Methyl-2-pyridylzinc bromide and the long reaction time (30hrs in order to order to reach 44% yield) needed in step b.

The advantage of the second method ("method 2" in scheme 2) - which is a further embodiment of the invention - is the use of cheaper, readily available and easy to handle reagents and much shorter reaction times, making this procedure more suitable for scale-up.

Accordingly, there is provided a method to obtain compound **D** that comprises the steps of:
a) treating compound **X3** with at least an equimolar amount of iodine and an excess of pyridine in a suitable solvent so as to obtain compound **X4**
b) treating compound **X4** with at least 1 equivalent methacrolein and an excess of an equimolar mixture of AcOH and CH₃COONH₄ in a polar solvent so as to obtain compound **D.**

Compound **X3** is commercially available or can be easily prepared from commercially available compounds by methods and reactions known to anyone skilled in the art.

Further research efforts have led to determine that ideal reaction conditions in which to perform step b) above are:5 eq AcOH, 5 eq CH₃COONH₄ and ethanol as a solvent.

Thus, in a further invention embodiment, compound **C** is prepared by:
a) treating compound **X3** with at least an equimolar amount of iodine and an excess of pyridine in a suitable solvent so as to obtain compound **X4**
b) treating compound **X4** with at least 1 equivalent methacrolein and an excess of an equimolar mixture of AcOH and CH₃COONH₄ in a polar solvent so as to obtain compound **D.**
c) treating compound **D** with at least 1eq isonipecotate, under palladium catalysis and in presence of a suitable base so as to obtain compound **X1**
d) converting compound **X1** into compound **X2**
e) coupling compound **X2** with N-methyl-piperazine so as to obtain compound **C.**

### Example 1: Synthesis route

### Synthesis of compound D

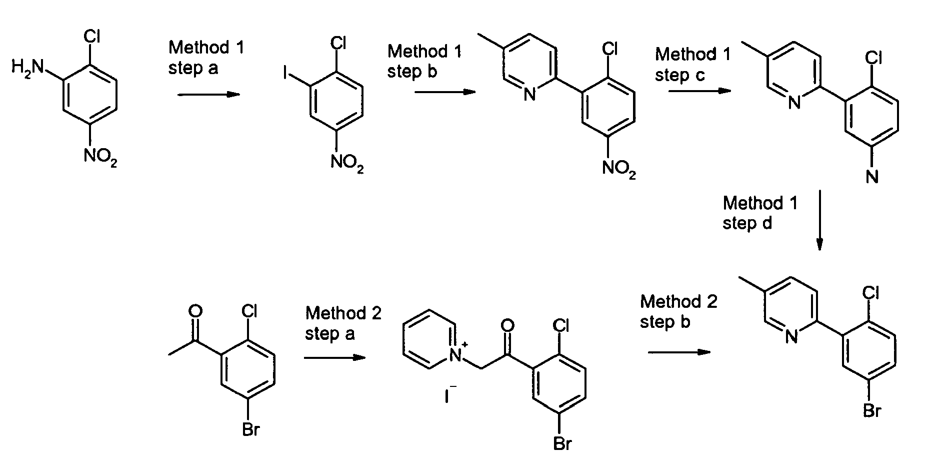

### 1-Chloro-2-iodo-4-nitro-benzene

**Method 1-Step a**-In a 3L four necked round bottom flask 2-chloro-5-nitrophenilamine (50.0 g, 289.7 mmol) was added to a solution of H₂O (800 ml) and conc. H₂SO₄ (41.0 ml, 405.6 mmol). The dark yellow suspension was cooled to 0°C and a solution of NaNO₂ (24.0 g, 347.6 mmol) in H₂O (100 ml) was added dropwise. The mixture was stirred 30 minutes at 0°C then a solution of KI (67.3 g, 405.6 mmol) in H₂O (300 ml) was added dropwise keeping the temperature below 10°C. The mixture was stirred 2h rt then checked by LC-MS. The suspension was extracted with EtOAc (4x800 ml), organic extracts were collected, washed with 10% Na₂S₂O₅ (2x1L) and brine (2x1L), then dried over MgSO₄, filtered and evaporated under reduced pressure to give 74.8 g of a crude brown solid. This was crystallized from iPrOH (200 ml) to give 58.1 g (204.9 mmol, yield 71%) of intermediate **(1)** as a brown-red crystalline solid (LC-MS assay >95%).

MS: not ionisable peak.

FTIR (cm⁻¹): 3086, 1522, 1342, 869, 738.

### 2-(2-Chloro-5-nitro-phenyl)-5-methyl-pyridine

**Method 1-step b**-In a 1L four necked round bottom flask, well dried under Ar flux, 1-chloro-2-iodo-4-nitro-benzene (1) (30.0 g, 105.8 mmol) was dissolved in anhydrous DMA (30 ml) then 5-methyl-2-pyridylzinc bromide (296.2 ml, 148.1 mmol), triphenylphosphine (5.6 g, 21.2 mmol) and tetrakis (triphenyl-phosphine) palladium(0) (6.1 g, 5.3 mmol) were added. The solution was heated to 60°C for 30 h, checking the progressive conversion by LC-MS. The reaction mixture was cooled to rt and added to a 1:1:1 EtOAc: NaOH 2M: crushed ice mixture (900 ml). The resulting mixture was stirred 1 h then left to stand 1 h and 30'. The brown suspension was filtered on a gooch washing the solid with EtOAc (300 ml). The filtrate was separated and the aqueous phase was extracted with EtOAc (3x400 ml). The collected organic extracts were washed with water (2x600 ml) and brine (2x600 ml) and concentrated to give a wet brown solid. This was taken up with HCl 1M (1L) and washed with EtOAc (2x500 ml). The organic layers were back-extracted with HCl 1M (2x500 ml) then the combined acid aqueous extracts were cooled to 0°C and made basic with NaOH 10M (450 ml). A brown solid was formed, this was filtered, washed with water (500 ml) and dried under vacuum (50°C) to give 11.6 g (46.6 mmol, yield 44%) of intermediate (2) as a brown solid (LC-MS assay 90%).

MS: m/z = 249/250 [M+H⁺]⁺; 266/267 [M+NH₄⁺]⁺.

FTIR (cm⁻¹): 1530, 1346, 1033, 886, 837, 739.

### 4-Chloro-3-(5-methyl-pyridin-2-yl)-phenylamine

**Method 1-step c**-In a 500 ml four necked round bottom flask 2-(2-chloro-5-nitro-phenyl)-5-methyl-pyridine_(11.6 g, 46.6 mmol) was suspended in EtOH (250 ml) then SnCl₂ (31.8 g, 167.8 mmol) and HCl 37% (37 ml) were added. The solution was heated to 60°C, stirred at this temperature for three hours and checked by LC-MS. Solvent was evaporated under reduced pressure and the residue was taken up with HCl 1M (500 ml) to give a suspension that was washed with EtOAc (3x300 ml). The aqueous layer was cooled to 0°C, made basic with NaOH 10M (120 ml) and extracted with EtOAc (2x600 ml). Combined organic layers were washed with Na₂CO₃ (2x500 ml), water (2x500 ml) and brine (2x500 ml), dried over MgSO₄, filtered and evaporated to give 7.8 g (35.7 mmol, yield 76%) of the desired compound as a brown oil (LC-MS assay >95%).

MS: m/z = 219/221 [M+H⁺]⁺.

### 2-(5-Bromo-2-chloro-phenyl)-5-methyl-pyridine (compound D)

**Method 1-step d**-In a 500 ml four necked round bottom flask a solution of NaNO₂ (2.7 g, 38.7 mmol) in water (12.2 ml) was added dropwise to a solution of 4-chloro-3-(5-methyl-pyridine-2-yl)-phenylamine (7.7 g, 35.2 mmol) in HBr 48% (12.3 ml) previously cooled to 0°C then the system was stirred 30 minutes rt. The reaction mixture was cooled to -5°C then a solution of CuBr (5.6 g, 38.7 mmol) in HBr 48% (8.4 ml) was added dropwising⁽¹⁾. The system was left to come to rt, stirred for one hour then checked by LC-MS. The reaction mixture was cooled to -5°C, made basic with NaOH 5N (100 ml) and extracted with EtOAc (5x150 ml). Collected organic layers were washed with water (3x200 ml) and brine (3x200 ml), dried over MgSO₄, filtered and concentrated under reduced pressure to give 8.5 g of crude product as a brown oil. This was purified by automatic column chromatography, to give 6.4 g (22.6 mmol, yield 64%) of product as a white solid (LC-MS assay >95%).

MS: m/z = 282/284/286 [M+H⁺]⁺.

FTIR (cm⁻¹): 3064, 2922, 1568, 1488, 1450, 1089, 1033, 1022, 827, 811,572,561.

¹H NMR (d6-DMSO): 2.40(s, 3H); 7.54(d, 1H); 7.62 (m, 2H); 7.73(m, 2H), 8.54(m, 1H).

### 1-[2-(5-Bromo-2-chloro-phenyl)-2-oxo-ethyl]-pyridinium iodide

**Method 2-Step a**-To a suspension of iodine (277 g, 1.1 mol) in iPrOAc (400 mL) in a 5 L 4-neck round bottom flask, cooled at 10°C, pyridine (433 mL, 5.35 mol) was added via dropping funnel in 5 min (ΔT= +5°C).

After complete addition a solution of 1-(5-Bromo-2-chloro-phenyl)-ethanone (250 g, 1.07 mol) in iPrOAc (600 mL) was via dropping funnel added at once (no exotherm observed). Further 300 mL of iPrOAc were added to wash the glassware and give the final reaction volume to 5 vol. The resulting mixture was heated at reflux until complete conversion of the acetophenone (18h from HPLC analysis).

The reaction mixture was then cooled to 15°C using an ice bath, filtered and the formed solid was washed with H₂O (1 L) and EtOH (450 mL). After filtration and drying until constant weight, 330 g of a yellow solid were obtained. Yield: 70%.

¹H-NMR (400 MHz DMSO-d6): δ 6.41 (2H, s), 7.64-7.66 (1H, m), 7.90-7.92 (1H, m), 8.28-8.13 (3H, m), 8.73-8.77 (1H, m), 8.98-8.99 (2H, m).

m/z 313 (M+H)⁺; retention time = 0.85/3 (HPLC).

### 2-(5-Bromo-2-chloro-phenyl)-5-methyl-pyridine (compound D)

**Method 2-Step b**-To a suspension of the 1-[2-(5-Bromo-2-chlorophenyl)-2-oxo-ethyl]-pyridinium iodide (330 g, 0.75 mol) in EtOH (2.2 L) in a 5 L 4-neck round bottom flask CH₃COONH₄ (289 g, 3.75 mol) was added portion wise (ΔT= -4°C). Then in sequence AcOH (215 mL, 3.75 mol) and a solution of methacrolein (93 mL, 1.13 mol) in EtOH (100 mL) were dropped (no exotherm detected) and the resulting mixture was heated at reflux until the complete consumption of the pyridinium salt (5h from HPLC analysis).

The reaction solution was concentrated under vacuum, the crude dissolved in DCM (1.2 L) and the organic phase washed with NaHCO₃ ss (500 mL), NaOH 15% (200 mL) and H₂O (400 mL) and the solvent then evaporated. A first trial of purification was done by dissolving the crude in iPrOH (1 L) in a 5 L 4-neck round bottom flask, heating at 45°C and adding slowly H₂O while maintaining the internal T~36°C while the crystallization was triggered by addition of crystal seed. The suspension was cooled at 15°C and stirred for 40 min, filtered and the solid washed with H₂O (500 mL). NMR analysis of the solid revealed a purity of 98%. The crude (~165 g) was suspended in cyclohexane (2 L) and the resulting suspension filtered and the solid washed with cyclohexane (100 mL). The mother liquors were transferred in a 5 L 4-neck round bottom flask, 8 g of activated charcoal were added and the resulting suspension was stirred at room T for 3h, filtered and the solvent evaporated. Finally 152 g of a yellow solid were obtained. Yield 71 %.

¹H-NMR (400 MHz CDC13): δ 2.32 (3H, s), 7.23-7.25 (1H, m), 7.33-7.36 (1H, m), 7.45-7.51 (2H, m), 7.67-7.68 (1H, m), 8.46-8.47 (1H, m).

*m*/*z* 283 (M+H)⁺; retention time = 2.45/5 (HPLC)

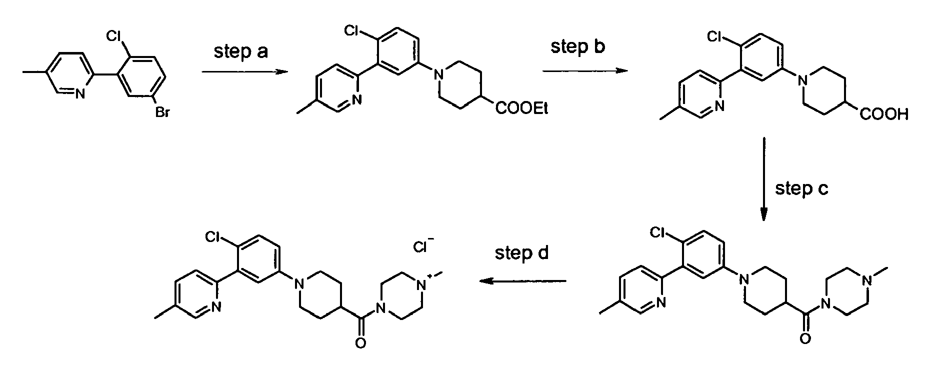

### 4-{1-[4-Chloro-3-(5-methyl-pyridin-2-yl)-phenyl]-piperidine-4-carbonyl}-1-methyl-piperazin-1-ium chloride

### 1-[4-Chloro-3-(5-methyl-pyridin-2-yl)-phenyl]-piperidine-4-carboxylic acid ethyl ester

Pd(OAc)₂ (12.4 g, 55.3 mmol), BINAP (35.3 g, 55.3 mmol) and toluene (2.6 L) were charged to 10 L jacketed reactor under nitrogen flux, and the suspension was stirred at 45°C for 20 min. Then ethyl isonipecotate (155 mL, 1 mol), 2-(5-Bromo-2-chloro-phenyl)-5-methyl-pyridine (260 g, 0.92 mol) and Cs₂CO₃ (900 g, 2.7 mol) were added respectively and the resulting mixture was heated at 110°C until complete conversion (2h from HPLC analysis).

The reaction mixture was cooled to room T, filtered with a Buchner funnel and the solid washed with EtOAc (1.4 L). The mother liquors were washed with H₂O (2 X 2 L) and NH₄Cl ss (2 L) and the solvent evaporated in order to obtain a brown oil (360 g) to be used for the next step without further purification.

*m*/*z* 359 (M+H)⁺; retention time = 1.56 (UPLC).

### 1-[4-Chloro-3-(5-methyl-pyridin-2-yl)-phenyl]-piperidine-4-carboxylic acid

To a suspension of the ester 7 (330 g, 0.92 mol) in 1,4-dioxane (2 L) in a 5 L 4-neck round bottom flask NaOH 15% (376 mL, 1.66 mol) was added drop-wise via dropping funnel in 5 min (ΔT= -4°C) and the resulting solution was heated at 80°C until the complete hydrolysis (3h from HPLC analysis).

The solvent was evaporated, the crude dissolved in H₂O (2 L) and the aqueous solution washed with iPrOAc (3 X 800 mL), acidized to pH 4.9 (measured by pH meter) and the formed suspension filtered with a Buchner funnel. The formed solid was washed with H₂O (1 L) and dried in oven (10 mbar, 60°C for 4h) giving 280 of a yellow solid (H₂O content 16.5% from Karl Fisher). The solid was then suspended in EtOH (2.2 L) in a 5 L 4-neck round bottom flask and the resulting suspension heated at reflux for 1h, cooled to room T, filtered with a Buchner funnel and the solid washed with EtOH (400 mL) and dried at rotavapor (4 mbar, 60°C for 1h) to furnish 210 g of a light yellow solid. Overall yield of 2 steps 69%.

*m*/*z* 331 (M+H)⁺; retention time = 1.12 (UPLC).

¹H-NMR (400 MHz DMSO-d6): δ 1.55-1.65 (2H, m), 1.84-1.88 (2H, m), 2.30-2.41 (4H, m), 2.73-2.80 (2H, m), 3.61-3.65 (2H, m), 6.96-7.02 (2H, m), 7.29-7.31 (1H, m), 7.49-7.51 (1H, m), 7.65-7.67 (1H, m), 8.48-8.49 (1H, m), 12.25 (1H, bp).

Karl Fisher: 0.5% of H₂O

### {1-[4-Chloro-3-(5-methyl-pyridin-2-yl)-phenyl]-piperidin-4-yl}-(4-methyl-piperazin-1-yl)-methanone (compound C)

To a nitrogen fluxed suspension of CDI (135 g, 828 mmol) in DCM (2.1 L) in a 5 L 4-neck round bottom flask 1-[4-Chloro-3-(5-methyl-pyridin-2-yl)-phenyl]-piperidine-4-carboxylic acid (210 g, 636 mmol) was added portion wise in 10 minutes. Intensive gas evolution, but no exotherm, was observed. The mixture was stirred at room temperature until complete activation of the acid (1h from HPLC analysis, quenching with butyl amine).

Then a solution of N-methyl-piperazine (71 g, 700 mmol) in DCM (80 mL) was added drop wise in 10 min (ΔT= +4°C) and the resulting mixture was stirred at room temperature for 3 days (98.5% of conversion). The reaction solution was washed with NaOH 0.9 M (4 X 1 L), dried over Na₂SO₄ and the solvent evaporated in order to obtain the title compound as a brown oil (270 g, 6.2% W/W DCM, HPLC purity 98%) to be used for the salification step without further purification.

*m*/*z* 413 (M+H)⁺; retention time = 0.84 (UPLC).

¹H-NMR (400 MHz DMSO-d6): δ 1.75-1.78 (2H, m), 1.88-1.98 (2H, m), 2.28 (3H, s), 2.35-2.39 (7H, s), 2.52-2.60 (1H, m), 2.70-2.77 (2H, m), 3.50-3.62 (4H, m), 3.70-3.73 (2H, m), 6.84-6.87 (1H, m), 7.08-7.09 (1H, m), 7.26-7.28 (1H, m), 7.49-7.54 (2H, m), 8.49 (1H, s).

### MATERIALS AND METHODS

### Method 1

LC-MS chromatograms were recorded on a Spectra System SCM100 chromatograph using a Zorbax Bonus RP (3.0 x 50 mm, I.D. 1.8 µm) and UV detection at 254 nm. The mobile phases consisted of 95% NH₄Ac (10 mM brought to pH 4 with HAc) with 5% MeOH as organic modifier. Flow rate is maintained at 1 ml/min.The concentration of the modifier was increased linearly from 5% to 95% over 7 min., the concentration of aqueous buffer was decreased linearly from 95% to 5% over 7 min. Then isocratic 95% MeOH for 7 min. Flow rate is maintained at 1 ml/min.The mass spectra of LC peaks were recorded using a Thermo Finnegan AQA single quadrupole spectrometer. HPLCs were recorded on a Perkin-Elmer HPLC-DAD system using a Phenomenex Gemini-NX c18 column (4.6 x 150 mm, I.D. 3.0 µm) and UV detection at 254 nm. The mobile phases consisted of 15% ammonium acetate buffer (10 mM brought to pH 4.2 with HAc) with 85% MeOH as organic modifier. Flow rate is maintained at 1 ml/min. ¹H-NMR spectra were recorded using a Varian Mercury 400 MHz spectrometer.FTIR were recorded on Jasco FT/IR-420Fourier transform infrared spectrometer.

Automatic column chromatography was performed on a Büchi MPLC system using silica gel Versaflash cartridges and characterized by a product-silica ratio ca. 1 g / 30 g, with EDP and ethyl acetate 9/1 mixture as eluent. Flow rate 0.5 CV/min.

### Method 2 and synthesis of the final compound

The reported yields are not corrected for purity and water/solvent content of the products.Generally, the reactions were monitored by HPLC and purities/conversions quoted refer to HPLC area% at 254 nm. HPLC conditions:

| | |
|---|---|
| Column Waters | Symmetry® C 18 3.5 µm 4.6x75 mm |
| Flow rate | 0.8 mL/min |
| Mobile phase A | 0.76% aq. K₂HPO₄ buffer or 0.1% aq. formic acid |
| Mobile phase B | acetonitrile |
| Gradient | 95:5 A/B to 20:80 A/B in 10 min, then 3 min |
| equilibration | |
| Over 3/5 minutes | |

Analytical UPLC -MS were run using a Acquity Waters UPLC with equipped with a Waters SQD (ES ionization) and Waters Acquity PDA detector, using a column BEH C18 1,7 µm, 2,1 x 5.00.

Gradient 0.1% formic acid/water and 0.1% formic acid/ CH3CN with a gradient 95/5 to 5/95 flow: 0.6 ml/min over 3 minutes

¹H-NMR spectra were recorded using a Varian Mercury 400 MHz spectrometer equipped with a PFG ATB Broadband probe. Humidity measurements were recorded on a Mettler Toledo V20.

### EXAMPLE 2: Compound C is a Smo receptor antagonist

The binding affinity of compound C with the Smo receptor was assessed using the competitive binding assay displacing fluorescently-labeled Bodipy-Cyclopamine described in WO2009074300, resulting in a Ki value below 100 nM.

The level of inactivation of the Hh pathway resulting from this binding was determined using the alkaline phosphatase-based assay described in WO2009074300, resulting in an IC50 value below 30 nM.

### EXAMPLE 3: Compound C has superior brain-permeating properties

The experimental design consists in the treatment of 3 CD-1 mice with the compound under study suitably formulated and PO administered at 5 mg/kg free base. For each sampling time (0.5,1.0 and 4.0 hours), plasma and brain are collected from the same animal and the resulting concentration data are used to provide information on the CNS partitioning by the calculation of the (AUC₀₋ₜ₍ₗₐₛₜ₎)_{bram} / (AUC₀₋ₜ₍ₗₐₛₜ₎)ₚₗₐₛₘₐ ratio, where AUC is the Area under Curve i.e., the geometric area of the trapezoid described by the concentration vs time plot.

Analysis based on LC-MS/MS after extraction was undertaken in plasma (7 levels, twice injected, ranging from 1 to 5000 ng.mL⁻¹). Plasma samples were treated only by protein precipitation (PP). Proteins were precipitated by addition of an organic solvent, acetonitrile (ACN) [volume ratio: 11 (organic solvent):1 (biological matrix)]. The resulting suspension was centrifuged at 3220g for 15min at 4°C. The supernatant was transferred in a suitable 96-well plate with calibrants and quality controls and then diluted with H₂O 0.1 % HCOOH before injection into the LC-MS/MS system.

Analysis based on LC-MS/MS and following dismembration and extraction with MeOH was undertaken in brain (8 levels, twice injected, ranging from 1 to 2000 ng.g⁻¹). Homogenisation of the brain tissue was performed with a Mikro-Dismembrator S (Sartorius). The brain, still frozen at +4°C, was cut by a scalpel in a Petri dish and transferred into a stainless steel shaking flask. After immersion in liquid nitrogen for 7 minutes, a tungsten carbide grinding ball was added, the cold chamber was quickly transferred to the Mikro Dismembrator for tissue homogenisation (2 minutes at 3000rpm). 50mg (±0.5 mg, allowing for an error of 1%) of the obtained powder were transferred to an Eppendorf tube and extracted by addition of an organic solvent, MeOH [volume ratio: 11 (organic solvent):1 (biological matrix)]. After vortexing for 5 min, samples were centrifuged for 30 min at 23755 g at +4°C. Supernatants were transferred to a 96-well plate with calibrants and quality controls for direct injection.

LC separation was performed for both plasma and brain samples on an UPLC Acquity System in fast gradient. The ESI⁺-MS/MS measurements are performed by a QTrap 5500 mass spectrometer (Applied Biosystems) in the multiple reaction monitoring (MRM) mode. Data were collected using Analyst 1.5. Both Q1 and Q3 were operating at unit resolution.

When tested under the above conditions, compounds A, B and C display brain-to-plasma partitioning values listed in table.

**Table**

| Compound | Vehicle | Brain: plasma partitioning AUC₀₋ₜ₍ₗₐₛₜ₎)_{brain} : (AUC₀₋ₜ₍ₗₐₛₜ₎)ₚₗₐₛₘₐ |
|---|---|---|
| | | |
| A | 50% PEG 400 and 50% Saline solution (NaCl 0.9%) v/v | 0.12 : 1 |
| B | PEG400 in saline solution 0.9% 30/70 (v/v) | 0.22 : 1 |
| C | PEG400 in saline solution 0.9% 30/70 (v/v) | 2.1 : 1 |

## Claims

1. The compound 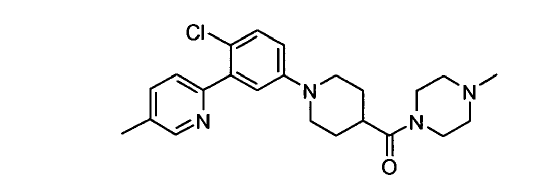 and pharmaceutically acceptable salts thereof.

2. The compound of claim 1 for use as a medicament.

3. The compound of claim 1 for use in the treatment of cancer, particularly in the treatment of a cancer selected from the list of: non-small cell lung carcinoma; small-cell lung cancer; breast cancer; ovarian tumours; digestive tract tumours; brain cancers; prostate cancer; pancreatic cancer; basal cell carcinoma; Gorlin syndrome; malignant melanoma; squamous cell carcinomas; multiple myeloma; lymphoma; mesenchymal cancers;chronic myeloid leukaemia; endometrial carcinoma; hepatocellular carcinoma.

4. The compound for use according to claim 3, wherein the cancer is selected from brain cancers.

5. The compound of claim 1 for use in the treatment of cancer metastases in the brain.

6. The compound for use according to claim 3, wherein the cancer is selected from a cancer that metastasises to the brain.

7. The use of the compound of claim 1 for the manufacture of a medicament, particularly for the manufacture of a medicament to treat cancer.

8. A method for preparing the compound of claim 1, **characterised in that** it comprises the steps of
a) treating compound **D** 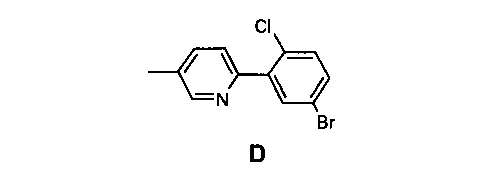 with at least 1eq isonipecotate, under palladium catalysis and in presence of a suitable base so as to obtain compound **X1** 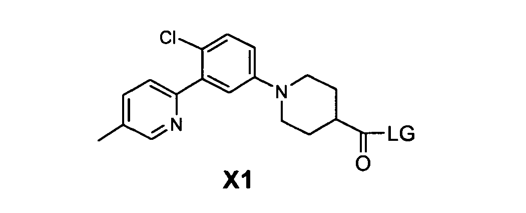 wherein LG is a suitable leaving group
b) converting compound **X1** into compound **X2** 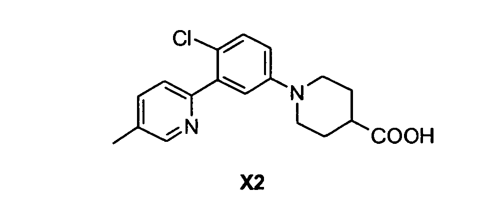
c) coupling compound **X2** with N-methyl-piperazine so as to obtain the compound of claim 1.

9. The method of claim 8, further comprising the following steps for preparing compound D:
a) treating compound **X3** 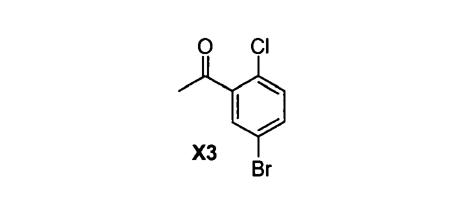 with at least an equimolar amount of iodine and an excess of pyridine in a suitable solvent so as to obtain compound **X4** 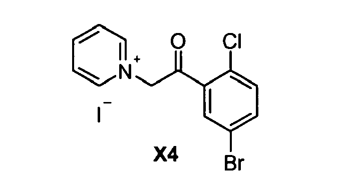
b) treating compound **X4** with at least 1 equivalent methacrolein and an excess of an equimolar mixture of AcOH and CH₃COONH₄ in a polar solvent so as to obtain compound **D**

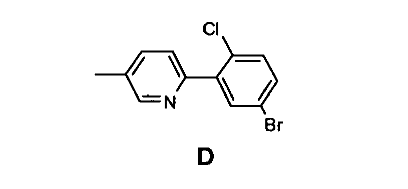

10. The method of claims 8 or 9, wherein LG is a linear branched or cyclic C₁₋₆ alkoxy group.

11. The compound

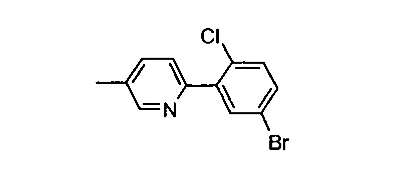

12. The use of compound of claim 11 as an intermediate or starting material in the preparation of the compound of claim 1.

## Patentansprüche

1. Verbindung 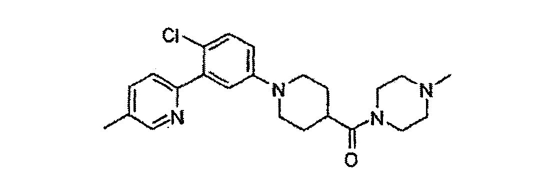 und pharmazeutisch annehmbare Salze davon.

2. Verbindung gemäß Anspruch 1 zur Verwendung als ein Medikament.

3. Verbindung gemäß Anspruch 1 zur Verwendung zur Behandlung von Krebs, speziell zur Behandlung einer Krebsart ausgewählt aus der Liste von: nicht-kleinzelligem Lungenkarzinom; kleinzelligem Lungenkarzinom; Brustkrebs; Gebärmutterhalstumoren; Tumoren des Verdauungstrakts; Gehirnkrebsarten; Prostatakrebs; Bauchspeicheldrüsenkrebs; Basalzellenkarzinom; Gorlin-Syndrom; malignem Melanom; squamösen Zell-Karzinomen; multiplem Myelom; Lymphomen; mesenchymalen Krebsarten; chronischer myeloischer Leukämie; endometrialem Karzinom; hepatozellulärem Karzinom.

4. Verbindung zur Verwendung gemäß Anspruch 3, wobei der Krebs ausgewählt ist aus Gehirnkrebsarten.

5. Verbindung gemäß Anspruch 1 zur Verwendung zur Behandlung von Krebs-Metastasen in dem Gehirn.

6. Verbindung zur Verwendung gemäß Anspruch 3, wobei der Krebs ausgewählt ist aus einem Krebs, der in das Gehirn metastasiert.

7. Verwendung der Verbindung gemäß Anspruch 1 zur Herstellung eines Medikaments, speziell für die Herstellung eines Medikaments um Krebs zu behandeln.

8. Verfahren zur Herstellung der Verbindung gemäß Anspruch 1, **dadurch gekennzeichnet, dass** es die Schritte umfasst
a) Behandeln von Verbindung D 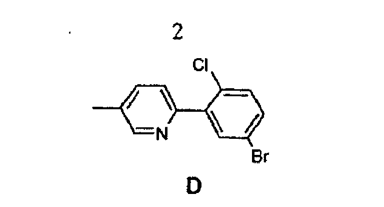 mit mindestens 1 Äquiv. Isonipectotat, unter Palladiumkatalyse, und in Anwesenheit einer geeigneten Base, um Verbindung **X1** zu erhalten 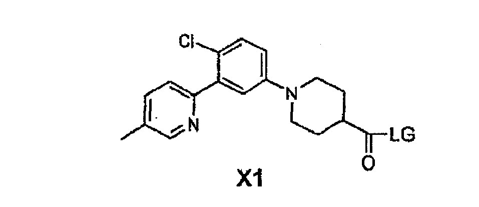 wobei LG eine geeignete Abgangsgruppe ist
b) Umwandeln von Verbindung **X1** in Verbindung **X2** 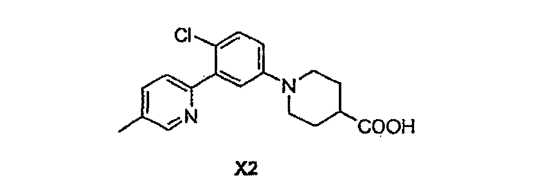
c) Koppeln von Verbindung **X2** mit N-Methyl-piperazin, um die Verbindung gemäß Anspruch 1 zu erhalten.

9. Verfahren gemäß Anspruch 8, das weiter die folgenden Schritte zur Herstellung von Verbindung D umfasst:
a) Behandeln von Verbindung **X3** 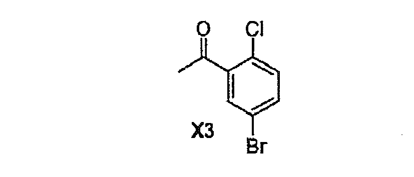 mit mindestens einer äquimolaren Menge an Iod und einem Überschuss an Pyridin in einem geeigneten Lösungsmittel, um Verbindung **X4** zu erhalten 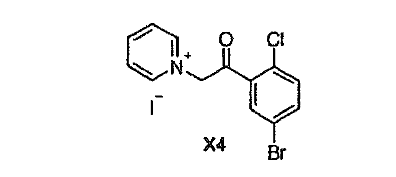
b) Behandeln von Verbindung **X4** mit mindestens 1 Äquivalent Methacrolein und einem Überschuss einer äquimolaren Mischung von AcOH und CH₃COONH₄ in einem polaren Lösungsmittel, um Verbindung D zu erhalten

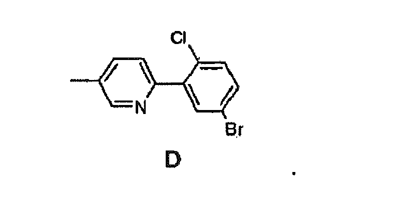

10. Verfahren gemäß Anspruch 8 oder 9, wobei LG eine lineare verzweigte oder cyclische C₁₋₆-Alkoxygruppe ist.

11. Verbindung

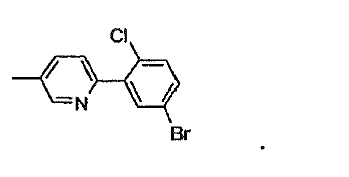

12. Verwendung einer Verbindung gemäß Anspruch 11 als ein Zwischenprodukt oder als Ausgangsmaterial zur Herstellung der Verbindung gemäß Anspruch 1.

## Revendications

1. Composé 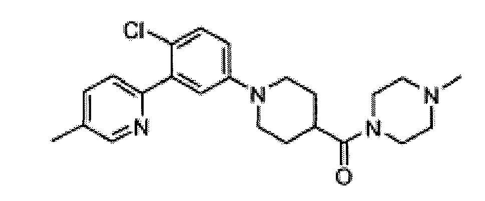 et les sels pharmaceutiquement acceptables de celui-ci.

2. Composé selon la revendication 1 pour l'utilisation comme médicament.

3. Composé selon la revendication 1 pour l'utilisation dans le traitement du cancer, particulièrement dans le traitement d'un cancer sélectionné parmi la liste du : carcinome du poumon non à petites cellules ; cancer du poumon à petites cellules ; cancer du sein ; tumeurs des ovaires ; tumeurs du tractus digestif ; cancers du cerveau ; cancer de la prostate ; cancer du pancréas ; carcinome des cellules basales ; syndrome de Gorlin ; mélanome malin ; carcinomes à cellules squameuses ; myélome multiple ; lymphome, cancers du mésenchyme ; leucémie myéloïde chronique ; carcinome de l'endomètre; carcinome hépatocellulaire.

4. Composé pour l'utilisation selon la revendication 3, dans lequel le cancer est sélectionné parmi les cancers du cerveau.

5. Composé selon la revendication 1 pour l'utilisation dans le traitement des métastases de cancer dans le cerveau.

6. Composé pour l'utilisation selon la revendication 3, dans lequel le cancer est sélectionné parmi un cancer qui produit des métastases au niveau du cerveau.

7. Utilisation du composé selon la revendication 1 pour la fabrication d'un médicament, particulièrement pour la fabrication d'un médicament pour traiter le cancer.

8. Procédé de préparation du composé selon la revendication 1, **caractérisé en ce qu'**il comprend les étapes de
a) traitement du composé **D** 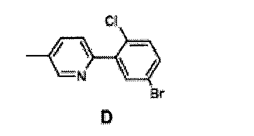 avec au moins 1 éq d'isonipécotate, sous catalyse au palladium et en présence d'une base appropriée afin d'obtenir le composé **X1** 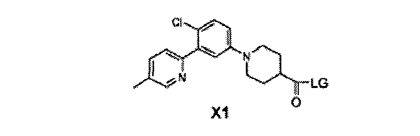 dans laquelle LG est un groupe partant approprié
b) conversion du composé **X1** en composé **X2** 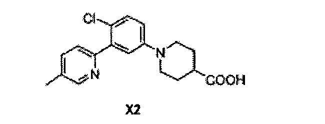
c) couplage du composé **X2** avec de la N-méthyl-pipérazine afin d'obtenir le composé selon la revendication 1.

9. Procédé selon la revendication 8, comprenant en outre les étapes suivantes de préparation du composé D :
a) traitement du composé **X3** 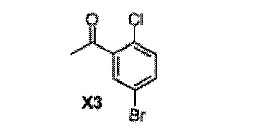 avec au moins une quantité équimolaire d'iode et un excès de pyridine dans un solvant approprié afin d'obtenir le composé **X4** 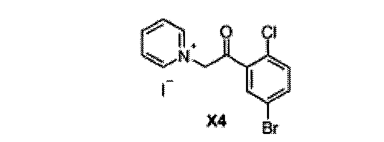
b) traitement du composé **X4** avec au moins 1 équivalent de méthacroléine et un excès d'un mélange équimolaire d'AcOH et CH₃COONH₄ dans un solvant polaire afin d'obtenir le composé **D**

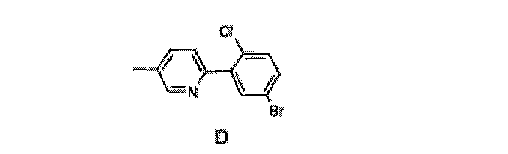

10. Procédé selon les revendications 8 ou 9, dans lequel LG est un groupe alcoxy en C₁-C₆ linéaire, ramifié ou cyclique.

11. Composé

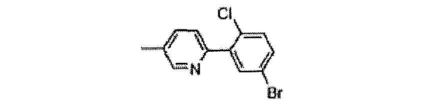

12. Utilisation du composé selon la revendication 11 comme intermédiaire ou matériau de départ dans la préparation du composé selon la revendication 1.
